## Europäisches Patentamt
## European Patent Office
## Office européen des brevets

(19)

(11) Numéro de publication: **0 194 931**
**B1**

(12) # FASCICULE DE BREVET EUROPÉEN

(45) Date de publication du fascicule du brevet:
15.11.89

(51) Int. Cl.⁴: **C 07 C 143/70, B 01 J 19/12**

(21) Numéro de dépôt: **86400467.6**

(22) Date de dépôt: **06.03.86**

(54) **Procédé et appareil pour la sulfochloration photochimique d'alcanes gazeux.**

(30) Priorité: **14.03.85 FR 8503744**
**03.03.86 FR 8602910**

(43) Date de publication de la demande:
**17.09.86 Bulletin 86/38**

(45) Mention de la délivrance du brevet:
**15.11.89 Bulletin 89/46**

(84) Etats contractants désignés:
**CH DE GB IT LI NL**

(56) Documents cité:
**FR-A-2 246 520**

(73) Titulaire: **SOCIETE NATIONALE ELF AQUITAINE (PRODUCTION), Tour Elf 2, Place de la Coupole La Défense 6, F-92400 Courbevoie (FR)**

(72) Inventeur: **Ollivier, Jean, Croix de Buzy, F-64260 Arudy (FR)**
Inventeur: **Baptiste, Hubert, 4 rue du Domaine du Chateau, F-64230 Lescar (FR)**
Inventeur: **Lagaude, Christian Henri, 1 rue Henri Dunant, F-64000 Pau (FR)**
Inventeur: **Larrouy, Michèle, 1 rue Henri Dunant, F-64000 Pau (FR)**

(74) Mandataire: **Kohn, Armand, 5 Avenue Foch, F-92380 Garches (FR)**

EP 0 194 931 B1

## Description

La présente invention se rapporte à la préparation photochimique de sulfochlorures d'alcanes; elle concerne plus particulièrement les synthèses de tels composés à partir d'alcanes gazeux à la température ambiante. L'invention comprend également un appareil pour la réalisation de cette synthèse.

Etant donné l'utilité industrielle des chlorures d'alcanes-sulfonyles, la préparation de ces corps a donné lieu à la mise au point de plusieurs procédés, dans le passé. L'action directe de $SO_2$ et $Cl_2$ sur un alcane, sous l'effet de l'ultraviolet, conduit à l'obtention de sulfochlorures, mais elle conduit à des mauvais rendements surtout à cause de la formation de fortes proportions de chlorure de sulfuryle. Comme la réaction exige l'évacuation de la chaleur de formation du composé voulu, il s'était avéré pratique d'opérer au sein d'un solvant. Ainsi le procédé classique consistait à faire barboter le mélange gazeux d'hydrocarbure, de chlore et d'anhydride sulfureux, dans un solvant et plus spécialement dans du tétrachlorure de carbone, le milieu réactionnel étant éclairé par la lumière ultraviolette. Dans ce procédé, qui est décrit dans l'ouvrage de F. ASINGER "Paraffines, Chemistry and Technology" (Pergamon Press 1968) p. 520 et suivantes, le solvant dissout le sulfochlorure formé, la chaleur de réaction est évacuée au moyen d'un circuit de refroidissement plongé dans le milieu réactionnel. Cependant, pour que la réaction au sein d'un solvant donne de bons résultats, il faut que celui-ci dissolve bien l'hydrocarbure à traiter; or, il est difficile de trouver un tel solvant, et celui qui convient le mieux industriellement, $CCl_4$ dissout bien les butanes et les propanes, mais déjà bien moins l'éthane et mal le méthane, même sous une pression de 8 bars, comme on peut le voir à la page 526 de l'ouvrage précité. En fait, le procédé classique de sulfochloration susindiqué ne donne pas des résultats satisfaisants avec le méthane, et ses rendements sont faibles dans le cas de l'éthane.

Un progrès important a été réalisé dans le procédé photochimique de sulfochloration par l'emploi d'une phase liquide dispersée, constituée par un liquide inerte, ne dissolvant pratiquement pas les réactifs, mais servant de milieu de contact entre eux. Un tel procédé est décrit dans la publication FR-2 246 520 qui indique l'acide sulfurique comme liquide inerte. Ce procédé permet d'éliminer convenablement la chaleur de réaction et obtenir de bons rendements sur le méthane; il comporte cependant la sujétion d'exiger de très forts débits de liquide, de l'ordre de 2 à 3 000 fois la production de chlorure de méthane sulfonyle produit.

La présente invention apporte sur la technique connue l'avantage de n'exiger l'introduction d'aucun produit étranger dans le milieu réactionnel et de former ce dernier uniquement avec ses constituants obligatoires, c'est-à-dire hydrocarbure, $SO_2$ et halogène, la réaction ayant lieu en phase gazeuse. Le procédé suivant l'invention permet l'obtention de très bonnes conversions et rendements satisfaisants tant sur l'hydrocarbure que sur l'halogène. En outre, le nouveau procédé se fait avec des rendements quantiques améliorés, parce qu'il contribue à une meilleure absorption des photons par l'halogène, alors que la chaleur de réaction est éliminée très aisément, évitant toute surchauffe du milieu gazeux réactionnel.

Le nouveau procédé s'applique bien à tous les alcanes gazeux à la température ambiante et ces avantages se font sentir tout particulièrement dans le cas du méthane, alcane le plus difficile à sulfochlorer selon la technique antérieure.

Le procédé suivant l'invention, qui consiste à faire réagir un alcane avec du $SO_2$ et un halogène, à une température modérée, en présence de la lumière ultraviolette, est caractérisé en ce que le mélange contient un fort excès de $SO_2$ par rapport à l'hydrocarbure et qu'en outre du $SO_2$ liquide est injecté dans la zone de réaction pour maintenir constante la température de celle-ci.

Dans une forme de réalisation de beaucoup préférée, le nouveau procédé est réalisé sous pression, telle que le mélange réactionnel reste à l'état gazeux, seul l'halogénure d'alcane sulfonyle subissant la liquéfaction.

Le procédé ainsi défini s'applique à tous les alcanes gazeux, à la température choisie pour la réaction, et à tous les halogènes: toutefois comme le sulfochlorure de méthane est très important industriellement, la suite de la présente description se rapporte surtout à ce corps.

Dans la réalisation du procédé suivant l'invention, les proportions préférées des réactifs, dans le mélange gazeux soumis au rayonnement ultraviolet, varient de préférence entre les limites suivantes:

| | par mole de $CH_4$ | par mole d'alcane en $C_2$ ou plus |
|---|---|---|
| | 4 à 8 moles $SO_2$ | 7 à 14 |
| | 0,4 à 1 mole $Cl_2$ | 0,6 à 1 |
| et mieux, | 5 à 7 moles $SO_2$ | 10 à 13 |
| | 0,7 à 0,9 mole $Cl_2$ | 0,7 à 0,9 |

Ces rapports sont aussi valables pour d'autres halogènes.

Le procédé peut être conduit à des températures d'environ 10° à 90°C avec une marge préférentielle de 30° à 70°C. Dans le cas du méthane, il est surtout avantageux de travailler entre environ 50° et 70°C ou mieux entre 55° et 65°C. Dans celui de l'éthane et des hydrocarbures supérieurs, des températures plus basses sont préférables, par exemple 30° et 40°C pour l'éthane. Comme déjà indiqué plus haut, la température voulue est réglée par l'injection de $SO_2$ liquide dans la phase gazeuse du réacteur.

La réalisation la plus avantageuse du nouveau procédé comporte une pression, au-dessus de la pression

EP 0 194 931 B1

atmosphérique, de 0,1 à 30 bars dans la phase gazeuse réagissante, et surtout de 8 à 15 bars. Bien entendu, cette pression est calculée en fonction de la température et de la composition du mélange réactionnel, pour que celui-ci reste gazeux.

Etant donné que la photo-sulfochloration des alcanes est connue en soi et qu'il est courant d'employer une lampe à vapeur de mercure pour la production du rayonnement ultraviolet utilisé, il n'est pas utile de décrire ici cette technique.

Le procédé suivant l'invention peut être mis en oeuvre dans une installation de type connu, mais celle-ci doit comporter certaines particularités qui sont indiquées sur le dessin annexé et dans sa description qui suit.

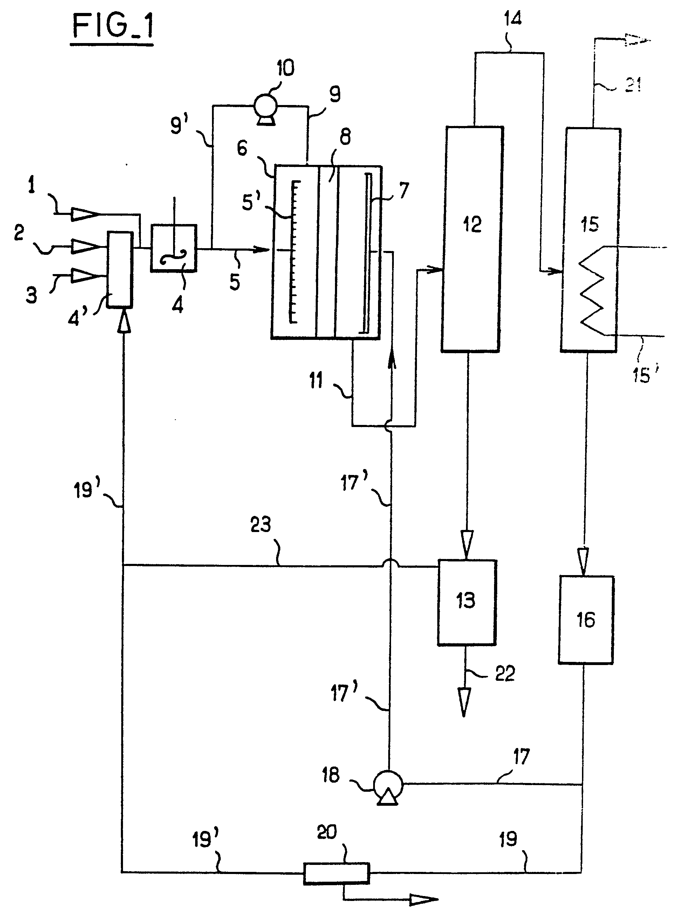

La figure 1 schématique représente un ensemble d'appareils pour la production d'alcanes sulfochlorés suivant le procédé de l'invention.

Sur le dessin, les entrées 1, 2 et 3 sont respectivement celles du méthane, du dioxyde de soufre et du chlore, gazeux, que l'on introduit dans un mélangeur 4 muni d'un agitateur pour homogénéiser le mélange gazeux. De préférence, un prémélangeur de $Cl_2$ avec $SO_2$ (repères 2 et 3) schématisé en 4', est prévu également pour des raisons de sécurité. Par la canalisation 5, le mélange gazeux passe du mélangeur 4 dans le réacteur 6, dans lequel il est distribué uniformément au moyen de la rampe 5', à orifices. Une autre rampe similaire 7 est placée également suivant la hauteur du réacteur pour servir à l'introduction du $SO_2$ liquide destiné au réglage de la température. Le réacteur est traversé à la manière connue en soi par une lampe à ultraviolet 8. Du haut du réacteur 6 part une canalisation 9 vers une pompe 10, permettant de recycler une fraction de l'effluent du réacteur vers la canalisation 5 en vue de la prédilution des réactifs venant de 4. La tubulure 11 conduit le produit liquide, formé dans le réacteur 6, vers le séparateur d'où la phase liquide, c'est-à-dire l'halogénure d'alcane sulfonyle brut, descend dans le stockage intermédiaire 13, tandis que les gaz résiduels passent par la conduite 14 dans le second séparateur 15; ce séparateur est éventuellement muni d'un refroidisseur pour ramener le $SO_2$, arrivant, à l'état liquide du $SO_2$ liquide, contenant du $Cl_2$, est recyclé après récupération dans un stockage intermédiaire 16; le recyclage du $SO_2$ liquide se fait par 17, par la pompe 18 et la canalisation 17' aboutissant à la rampe 7 dans le réacteur 6. Une autre fraction de $SO_2$, venant de 16, passe par la canalisation 19 dans l'évaporateur 20 et de là par 19' vers l'alimentation du mélangeur 4.

En haut du séparateur 15 est évacué le HCl par la conduite 21 vers des appareils de traitement non représentés.

Du bas du stockage intermédiaire 13 part une conduite 22 vers des appareils de purification de l'halogénure d'alcane sulfonyle produit qui, ne faisant pas l'objet de l'invention, ne sont pas représentés ici.

La principale caractéristique, qui distingue l'appareil suivant l'invention de ceux de la technique antérieure, réside en ce que des moyens 7 de distribution de liquide sont placés dans le réacteur 6 parallèlement à la lampe à ultraviolet 8. De tels moyens, qui pratiquement se réduisent à une rampe à perforations, sont bien plus faciles à loger dans le réacteur que ne le sont des réfrigérants plongés dans la phase liquide d'un réacteur, de l'art antérieur. En outre, l'échange de chaleur par contact direct du $SO_2$ avec le mélange gazeux est beaucoup plus efficace que l'échange à travers la paroi d'un faisceau tubulaire ou d'un serpentin de refroidissement plongé dans le milieu réactionnel, selon la technique antérieure.

Une autre caractéristique de l'installation suivant l'invention est un mélangeur 4 où se prépare le mélange des réactifs gazeux destinés à la réaction dans l'enceinte 6. Dans une installation perfectionnée, un premier mélangeur 4' sert à mélanger $Cl_2$ avec $SO_2$, et le prémélange, ainsi formé, rencontre l'hydrocarbure dans 4. Suivant une disposition préférée, sur le passage 5 de 4 à 6 est branchée une arrivée 9' d'une fraction de gaz provenant du réacteur 6, ce qui constitue également une caractéristique nouvelle de l'appareillage.

Bien que toute installation de ce type comporte obligatoirement un séparateur 12 qui reçoit le liquide formé dans le réacteur, l'installation suivant l'invention présente encore une caractéristique nouvelle en ce qu'elle comporte un second séparateur 15 fonctionnant à une température telle que $SO_2$ liquide s'écoule dans le stockage intermédiaire 16 pour être, de là, acheminé dans les moyens d'injection 7 dans le réacteur 6. En haut du séparateur 15 la quasi totalité de l'hydracide formé est dégagé par 21 pour aller vers des appareils appropriés.

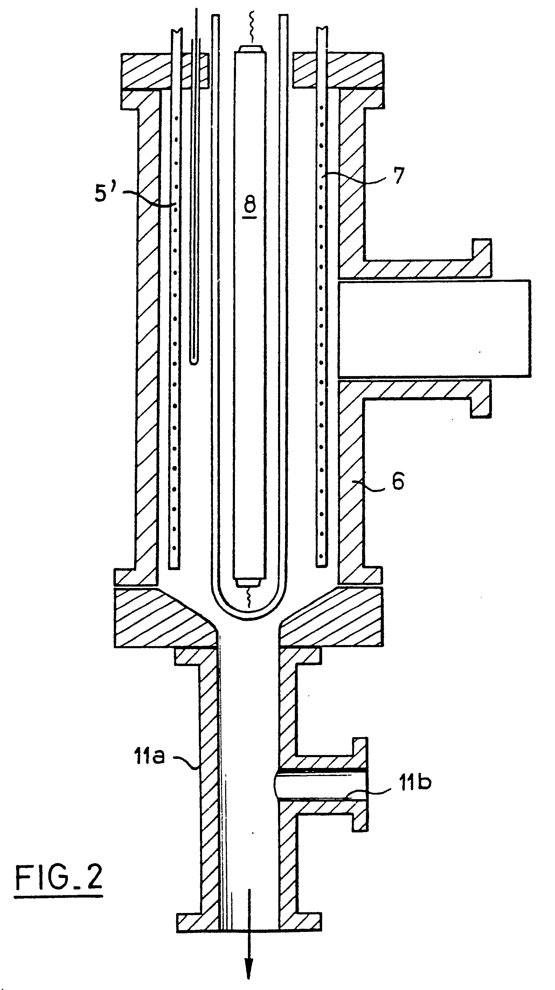

La figure 2 représente en coupe axiale le réacteur 6 équipé d'une rampe 5' de distribution du mélange réactionnel, d'une rampe 7 pour l'injection de $SO_2$ liquide, et d'une lampe à ultraviolet 8. Au lieu d'une sortie 11 allant vers le premier séparateur 12, comme dans la figure 1, on a ici une sortie 11a verticale, conduisant le liquide du fond du réacteur directement au stockage intermédiaire 13, alors qu'un embranchement 11b est relié au séparateur 12. Il suffit d'une colonne suffisamment haute entre 11b et 13 pour que l'halogénure d'alcane sulfonyle liquide, produit en 6, s'écoule régulièrement de 11a à 13, et que les gaz s'échappent par 11b vers 12.

En fonctionnement de l'installation suivant l'invention, le méthane arrive par 1, le dioxyde de soufre par 2 et le chlore par 3. Ces gaz sont mélangés en 4' et 4 et acheminés par 5 dans le réacteur 6. De préférence, une fraction de gaz du réacteur 6 est introduite par 9, 10, 9' dans le courant gazeux passant par 5, ce qui produit une prédilution du mélange gazeux. Ces réactifs sont distribués suivant la hauteur du réacteur au moyen d'une rampe 5' qui produit une diffusion homogène dans le réacteur.

La lampe à mercure 8 étant allumée, et la température de l'intérieur du réacteur réglée à la valeur voulue par l'injection de $SO_2$ liquide à l'aide de la rampe 7, la réaction a lieu et l'halogénure d'alcane sulfonyle produit s'écoule du bas du réacteur par 11, pour être acheminé dans le séparateur 12.

La phase liquide, qui s'écoule du séparateur 12 dans le stockage intermédiaire 13, est constituée par l'halogénure d'alcane sulfonyle brut elle peut donc contenir certaines proportions de produits secondaires, notamment du dichlorure de méthylène $CH_2Cl_2$, de chloroforme $CHCl_3$, de tétrachlorure de carbone $CCl_4$,

3

d'hexachloroéthane $C_2Cl_6$, et de diméthyldisulfone $CH_3SO_2SO_2CH_3$. Le procédé suivant l'invention permet d'avoir très peu de ces impuretés, néanmoins, la purification par distillation s'impose à la manière connue, ce qui a lieu dans un circuit auquel conduit la canalisation 22.

La récupération de l'anhydride sulfureux sous forme liquide, accompagné du chlore résiduel, a lieu dans le second séparateur 15. Ainsi, suivant l'invention, possède-t-on un stock de $SO_2$ liquide dans un stockage intermédiaire 16, d'où une partie de ce composé passe par 17 vers les moyens de refroidissement 7 du réacteur 6, et une autre partie, par 19, vaporisé en 20, se rend par la canalisation 19' à l'entrée du mélangeur 4. Donc l'excès de $SO_2$, employé suivant l'invention, est continuellement recyclé, d'une part à l'état liquide pour le refroidissement du mélange réactionnel en 6, et d'autre part à l'état gazeux vers le mélange initial en 4 et 4'.

Comme on vient de le voir, le $SO_2$ recyclé ne contient pratiquement pas d'hydracide résiduel, en particulier HCl, du fait de la bonne capacité de séparation de la colonne 15. Quant au $SO_2$ dissous dans le produit liquide de la réaction, il peut être dégazé à partir de 13 et récupéré par la conduite 23.

**Exemple 1**

Le procédé et l'appareil suivant l'invention ont été appliqués, à titre d'exemple, à la préparation de chlorure de méthane sulfonyle $CH_3SO_2Cl$. Dans un réacteur 6 de 6,5 litres de capacité, contenant une lampe à vapeur de mercure de 15 watts placée axialement, la préparation suivante a été effectuée.

Le mélange gazeux, préparé en 4, contient pour 1 mole de $CH_4$ 6,25 moles de $SO_2$ et 0,83 mole de $Cl_2$.

Le débit horaire de ce gaz d'alimentation est de 250 litres N/heure. La pression dans le réacteur est fixée à 4 bars au-dessus de l'atmosphère. La température est réglée à 60° ± 2°C par injection de $SO_2$ liquide au moyen de la rampe 7: on introduit ainsi 200 g de $SO_2$/heure.

Dans le stockage 13 on recueille par heure 125 g de chlorure de méthane sulfonyle brut après détente. A la pression atmosphérique et à la température ambiante, ce produit présente la composition suivante:

| | |
|---|---|
| $CH_3SO_2Cl$ | 75 % |
| $SO_2$ | 20 |
| $CH_3Cl$ | 1,6 |
| $CH_2Cl_2$ | 1,2 |
| $CHCl_3$ | 1,2 |
| Produits lourds | 1 |

L'effluent gazeux, qui arrive par 14 dans le 2ème séparateur 15, présente la composition en volume:

| | |
|---|---|
| $SO_2$ | 80 % |
| HCl | 14 |
| $Cl_2$ | 1,2 |
| $CH_4$ | 2,9 |
| $CH_3Cl$ | 1,9 |

Afin de recueillir le $SO_2$ à l'état liquide sous 4 bars de pression relative, on maintient la température dans le séparateur 15 au-dessous de 32°C.

Le débit de $CH_4$ à la sortie 21 du séparateur 15 est de 7,16 litres N/heure contre 29,4 introduits en 1; la conversion de méthane s'élève donc à 76 %. Pour le chlore, la conversion est de 88 %. Les résultats de cet essai conduisent aux rendements et sélectivités suivants en chlorure de méthane sulfonyle produit:

| | Rendement % | Sélectivité % |
|---|---|---|
| sur le méthane | 62,3 | 82,4 |
| sur le chlore | 75,2 | 85,5 |

**Exemple 2**

Préparation de chlorure d'éthane sulfonyle

Le procédé et l'appareil, décrits plus haut, ont été utilisés à la préparation du chlorure d'éthane sulfonyle $CH_3CH_2SO_2Cl$.

Comme l'halogénation d'un alcane est d'autant plus rapide que ce dernier possède une structure moins symétrique, l'éthane se chlore plus vite que le méthane, et il est plus sensible aux fortes concentrations en chlore. C'est pourquoi, dans le présent exemple le mélange gazeux employé est plus dilué avec $SO_2$ que celui de l'exemple 1; pour 1 mole de $C_2H_6$ il contient 12,5 moles de $SO_2$ et 0,83 mole de $Cl_2$.

4

Le débit horaire du gaz d'alimentation est de 327 N litres/heure.

La pression dans le réacteur est fixée à 4 bars au-dessus de la pression atmosphérique. Pour limiter les halogénations, on a constaté qu'une température de 40 ± 5°C est préférable. On introduit $SO_2$ liquide au moyen de la rampe 7, pour ajuster la température; la quantité ainsi injectée est de 165 g $SO_2$/heure.

Dans le stockage 13 on recueille par heure 113,8 g de chlorure d'éthane sulfonyle brut, le tout après détente. A la pression atmosphérique et la température ambiante, ce produit présente la composition suivante:

| | |
|---|---|
| $CH_3CH_2SO_2Cl$ | 75 % |
| $SO_2$ | 20 % |
| produits halogénés divers | 5 % |

L'effluent gazeux, qui arrive dans le 2ème séparateur 15, présente la composition en volume:

| | |
|---|---|
| $SO_2$ | 88,4 % |
| HCl | 4,6 % |
| $Cl_2$ | 0,5 % |
| $C_2H_6$ | 2,0 % |
| chlorés | 4,5 % |

La température dans le séparateur 15 est maintenue inférieure à 32°C.

Le débit de $C_2H_6$ à la sortie 21 du séparateur 15 est de 7,0 N l/h contre 24,42 introduits en 1: la conversion de l'éthane s'élève donc à 71,3 %. Pour le chlore, la conversion est de 91 %. Les résultats de cet essai conduisent aux rendement et sélectivité suivants en éthane sulfochlorure

| | Rendement % | Sélectivité % |
|---|---|---|
| Sur l'éthane | 61 % | 85 % |
| Sur le chlore | 73 % | 80 % |

Le procédé est également bien adapté à la synthèse d'isomères sulfochlorés du propane et du butane. On produit alors deux isomères de chacun des monosulfochlorures dérivés de ces alcanes.

**Exemple 3**

Préparation du propane sulfochlorure

Le mélange gazeux a une composition molaire identique à celle de l'exemple 2. Le débit horaire d'alimentation est de 327 N l/h. On fixe la pression à 6 bars au-dessus de la pression atmosphérique et la température à 50°C. Pour fixer la température on introduire 200 g de $SO_2$ liquide à l'heure.

Dans le stockage 13 on recueille par heure 127,4 g de chlorure de propane sulfonyle brut, après détente. A la pression atmosphérique, et à la température ambiante, ce produit contient 85 % en poids du mélange des deux isomères sulfochlorés du propane, soit 108,3 g.

La répartition des deux isomères est la suivante:

| | |
|---|---|
| $CH_3\text{-}CH_2\text{-}CH_2\text{-}SO_2Cl$: | 71,4 g (66 %) |
| $CH_3\text{-}CH\text{-}CH_3$: | 36,9 g (34 %) |
| $\quad\quad\;\; \mid$ | |
| $\quad\quad SO_2Cl$ | |

soit sensiblement 2/3 de l'isomère-1 pour 1/3 de l'isomère-2.

Ces chiffres sont issus de l'analyse chromatographique et de l'étude par résonance magnétique nucléaire.

La préparation a donné un rendement de:

70 % par rapport au propane
84 % par rapport au chlore

pour l'ensemble des deux isomères.

Les autres produits formés sont des isomères mono et poly chlorés du propane.

## Exemple 4

### Préparation du butane sulfochlorure

La composition molaire du mélange gazeux à l'entrée du réacteur est celle des exemples 2 et 3, ainsi que le débit de 327 N l/h. Par contre, pour être certain de faire réagir les réactifs sous forme de vapeur, la pression totale est de 5 bars et la température de 60°C. 150 g de $SO_2$ liquide à l'heure sont suffisants pour maintenir la température au niveau désiré.

La production brute des deux isomères sulfochlorés du butane est de 135 g/h. Le produit est pur à environ 85 %, ce qui donne une production de 114,7 g/h répartis en:

$CH_3-CH_2-CH_2-CH_2-SO_2Cl$:      64,9 g (57 %)

$CH_3-CH_2-CH-CH_3$:      49,8 g (43 %)
       |
    $SO_2Cl$

Le rendement global est de 81 % par rapport au chlore et de 67,24 % par rapport au butane.

## Exemple 5

### Préparation de chlorure de méthane sulfonyle $CH_3SO_2Cl$ (désigné par l'abréviation CMS).

On opère comme dans l'exemple 1 du brevet principal, mais sous une pression absolue de 10 bars et à une température de 50°C dans le réacteur.

Le mélange gazeux, mis en oeuvre, contient pour une mole de $CH_4$ 5,41 moles de $SO_2$ et 0,71 mole de $Cl_2$, pour un débit horaire de 233. La pression partielle du $SO_2$ dans le réacteur est de 7,6 N l/h.bars.

Dans le stockage 13, maintenu à une température de 120°C on recueille par heure 122 g d'un mélange contenant du CMS, des dérivés halogénés du méthane et du $SO_2$ dissous, selon la composition suivante:

| | |
|---|---|
| $-CH_3SO_2Cl$ | 73,3 % |
| $SO_2$ | 23,3 % |
| $CH_3Cl$ | - |
| $CH_2Cl_2$ | 1,03 % |
| $-CHCl_3$ | 0,96 % |
| $-CCl_4$ | 0,25 % |
| Produits lourds | 1,15 % |

L'effluent gazeux, qui arrive par 14 dans le 2ème séparateur 15 présente la composition en volume:

| | |
|---|---|
| $-SO_2$ | 85,1 % |
| $-HCl$ | 8,34 % |
| $-Cl_2$ | 0,97 % |
| $-CH_4$ | 4,72 % |
| $-CH_3Cl$ et autres | 0,87 |

On recueille le $SO_2$ après détente à 6 bars, comme dans l'exemple 1.

La conversion du méthane s'élève à 63 %. Pour le chlore, la conversion est de 90 %. Les résultats de cet essai conduisent aux rendements et sélectivités suivants en chlorure de méthane sulfonyle produit:

| | Rendement % | Sélectivité % |
|---|---|---|
| Sur le méthane | 54 | 86 |
| Sur le chlore | 76 | 85,2 |

## Exemple 6

Dans cet exemple la pression totale est de 10 bars absolus et le mélange gazeux contient, pour une mole de $CH_4$, 14,7 moles de $SO_2$ et 0,7 mole de $Cl_2$; la pression partielle de $SO_2$ est donc de 9 bars. Débit horaire 536,2 N l/h.

On recueille par heure 110,6 g de CMS brut de composition:

6

EP 0 194 931 B1

| CH₃SO₂Cl | 77 % |
|---|---|

$CH_3SO_2Cl$ 77 %
$SO_2$ 21,51 %
$CH_2Cl_2$ 0,61 %
$CHCl_3$ 0,87 %

L'effluent gazeux qui arrive dans le second séparateur:

$SO_2$ 94,9 %
$HCl$ 3,2 %
$Cl_2$ 0,8 %
$CH_4$ 0,9 %
$CH_3Cl$ 0,025 %
Autres 0,17 %

Les résultats de cet essai sont les suivants, avec une conversion en chlore de 81 %:

| | Rendement % | Sélectivité % |
|---|---|---|
| Sur le méthane | 51 | 85 |
| Sur le chlore | 73 | 90 |

**Exemple 7**

Mêmes conditions générales que dans l'exemple 5 mais sous une pression totale de 15 bars; pression partielle de $SO_2$ 11,4 bars. La température est maintenue à 65°C par injection de 200 grammes à l'heure de $SO_2$ liquide.
On recueille par heure, en 13, 154 g de CMS brut de composition suivante:

$CH_3SO_2Cl$ 63,1 %
$SO_2$ 35,2 %
$CH_2Cl_2$ 0,74 %
$CHCl_3$ 0,51 %

Dans l'effluent gazeux, qui arrive par 14 dans le 2ème séparateur, on a:

$SO_2$ 85,4 %
$HCl$ 8,3 %
$Cl_2$ 0,9 %
$CH_4$ 5,1 %
$CH_3Cl$ 0,03 %
Autres 0,25 %

**Exemple 8**

Les opérations de l'exemple 1 sont répétées dans les conditions suivantes:

4,22 moles $SO_2$ et 0,73 mole $Cl_2$ par mole de $CH_4$;
Pression absolue 10 bars, température 50 ± 2°C
Débit de gaz 250 litres N/heures.
On recueille 183,6 g de liquide par heure dans le stockage 13.

La température, dans le séparateur 15, est maintenue au-dessous de 38°C et la pression à 7 bars, afin que le $SO_2$ y soit liquide.
La conversion du $CH_4$ est de 76 %, celle du $Cl_2$ 86 %.
Rendement: sur $CH_4$ 63 %, sur $Cl_2$ 75 %;
Sélectivité: sur $CH_4$ 83 %, sur $Cl_2$ 87 %.

7

## Exemple 9

On répète l'exemple 8 mais sous une pression de 1,6 bars, à 50°C. Les rendements sont alors de 40 % sur $CH_4$ et 47,6 % sur $Cl_2$ et les sélectivités correspondantes respectivement 52,7 % et 55,2 %.

Récapitulation des exemples 1 et 5 à 9

Aux fins de comparaison, on reprend ici les résultats de quelques exemples. P désigne la pression absolue. $P_{SO_2}$ désigne la pression partielle de $SO_2$ dans le mélange gazeux, réagissant.

| | Exemple n° | | | | | |
|---|---|---|---|---|---|---|
| | 1 | 5 | 6 | 7 | 8 | 9 |
| P totale (réacteur) | 5 | 10 | 10 | 15 | 10 | 1,6 |
| $P_{SO_2}$ (réacteur) | 4,8 | 7,6 | 9 | 11,4 | 7,1 | 1,4 |
| Température °C | 60° | 60° | 60° | 65° | 50° | 50° |
| $SO_2/CH_4$ molaire | 6,2 | 5,4 | 14,7 | 5,4 | 4,2 | 4,2 |
| Rendement sur $CH_4$ % | 62,3 | 54 | 51 | 68 | 63 | 40 |
| Sélectivité sur $CH_4$ % | 82,4 | 86 | 85 | 94 | 83 | 52,7 |
| Sélectivité sur $Cl_2$ % | 85,5 | 85,2 | 90 | 90 | 87 | 55,2 |

On peut constater que la sélectivité et le rendement sur $CH_4$ en chlorure de méthane sulfonyle croissent avec la pression du mélange gazeux dans le réacteur; ainsi, de 82,4 % pour 5 bars (exemple 1) la sélectivité arrive à 94 % avec 15 bars (exemple 7). Mais il est préférable que le nombre de moles de $SO_2$, présentes par mole de $CH_4$, soit de l'ordre de 5 à 7, parce qu'un nombre trop élevé, voisin de 15 (exemple 6), conduit à des rendements et sélectivités quelque peu réduites.

Il est à noter également que plus la pression est élevée, meilleure est l'élimination de HCl dans le séparateur 15.

## Revendications

1. Procédé de préparation d'halogénures d'alcane sulfonyles par la réaction entre un alcane, du dioxyde de soufre et un halogène, en présence de la lumière ultraviolette, en phase gazeuse, caractérisé en ce que le mélange gazeux des réactifs contient au moins 4 moles de $SO_2$ par mole de l'hydrocarbure.

2. Procédé suivant la revendication 1, caractérisé en ce que la température dans la zone de réaction est réglée par l'injection de $SO_2$ liquide dans le mélange gazeux réagissant.

3. Procédé suivant la revendication 1 ou 2, caractérisé en ce que la réaction a lieu à une température de 10 à 90°C, sous une pression de 0,1 à 30 bars, telles que le mélange réagissant reste gazeux.

4. Procédé suivant la revendication 3, caractérisé en ce que la température est comprise entre 30° et 70°C et la pression relative entre 7 et 15 bars.

5. Procédé suivant une des revendications précédentes, dans lequel l'alcane est le méthane, la température étant de 50 à 70°C.

6. Procédé suivant une des revendications précédentes, caractérisé en ce que le mélange réactionnel contient, par mole de méthane, 4 à 8 moles de $SO_2$ et 0,6 à 1 mole de $Cl_2$ ou par mole d'un autre alcane gazeux 7 à 14 moles de $SO_2$ et 0,6 à 1 mole $Cl_2$.

7. Procédé suivant la revendication 6, caractérisé en ce que les proportions, par mole de méthane, sont de 5 à 7 moles $SO_2$ et 0,7 à 0,9 mole $Cl_2$, ou par mole d'un autre alcane gazeux 10 à 13 moles $SO_2$ et 0,7 à 0,9 mole $Cl_2$.

8. Appareil pour la réalisation du procédé suivant une des revendications 1 à 7, qui comporte un mélangeur (4) de réactifs gazeux alimentant un réacteur (6) comportant une lampe à ultraviolet (8) et des séparateurs (12, 13) en aval du réacteur (6), caractérisé en ce que le réacteur (6) est muni des moyens (7) pour l'injection (16, 17, 18,17') de $SO_2$ liquide dans le réacteur.

9. Appareil suivant la revendication 8, caractérisé en ce que le mélangeur (4) est précédé d'un prémélangeur (4') pour le $SO_2$ (2) et le chlore (3).

10. Appareil suivant la revendication 8 ou 9, caractérisé en ce qu'il est muni d'un séparateur (15) supplémentaire, équipé des moyens de refroidissement (15') pour la condensation du $SO_2$ excédentaire, provenant du réacteur (6) et du premier séparateur (12).

11. Appareil suivant la revendication 10, caractérisé en ce qu'il comprend un évaporateur (20) et une canalisation de recyclage (19, 19') pour réutiliser dans le prémélangeur (4') une partie du $SO_2$ liquéfié (15, 16).

12. Appareil suivant une des revendications 8 à 11, caractérisé en ce qu'il comporte un circuit (9, 10, 9') pour réintroduire une fraction du produit brut, formé dans le réacteur, dans la conduite (5) menant le mélange gazeux vers ce réacteur.

13. Appareil suivant une des revendications 8 à 12, caractérisé en ce que le fond du réacteur (6) est relié directement à un stockage (13) situé à un niveau inférieur, par une conduite (11a) dont la partie supérieure porte un embranchement (11b) vers un séparateur de gaz (12).

## Patentansprüche

1. Verfahren zur Herstellung von Alkansulfonylhalogeniden durch Umsetzung eines Alkans, von Schwefeldioxid und eines Halogens in Gegenwart von ultraviolettem Licht in Gasphase, dadurch gekennzeichnet, dass das Gasgemisch der Reaktionsteilnehmer mindestens 4 Mol $SO_2$ pro Mol Kohlenwasserstoff enthält.

2. Verfahren nach Anspruch 1, dadurch gekennzeichnet, dass die Temperatur in der Reaktionszone durch Einspritzen von flüssigem $SO_2$ in das reagierende Gasgemisch eingestellt wird.

3. Verfahren nach Anspruch 1 oder 2, dadurch gekennzeichnet, dass die Reaktion bei einer Temperatur von 10 bis 90°C und einem Druck von 0,1 bis 30 bar stattfindet, so dass das reagierende Gemisch gasförmig bleibt.

4. Verfahren nach Anspruch 3, dadurch gekennzeichnet, dass die Temperatur zwischen 30 und 70°C und der relative Druck zwischen 7 und 15 bar liegen.

5. Verfahren nach einem der vorstehenden Ansprüche, bei dem es sich beim Alkan um Methan handelt, wobei die Temperatur 50 bis 70°C beträgt.

6. Verfahren nach einem der vorstehenden Ansprüche, dadurch gekennzeichnet, dass das Reaktionsgemisch pro Mol Methan 4 bis 8 Mol $SO_2$ und 0,6 bis 1 Mol $Cl_2$ oder pro Mol eines anderen gasförmigen Alkans 7 bis 14 Mol $SO_2$ und 0,6 bis 1 Mol $Cl_2$ enthält.

7. Verfahren nach Anspruch 6, dadurch gekennzeichnet, dass die Verhältnisse pro Mol Methan 5 bis 7 Mol $SO_2$ und 0,7 bis 0,9 Mol $Cl_2$ oder pro Mol eines anderen gasförmigen Alkans 10 bis 13 Mol $SO_2$ und 0,7 bis 0,9 Mol $Cl_2$ betragen.

8. Vorrichtung zur Durchführung des Verfahrens nach einem der Ansprüche 1 bis 7, das einen Mischer (4) für die einem Reaktor (6) zugeführten gasförmigen Reaktionsteilnehmer aufweist, wobei eine ultraviolette Lampe (8) und Separatoren (12, 13) stromabwärts zum Reaktor (6) vorgesehen sind, dadurch gekennzeichnet, dass der Reaktor (6) mit Mitteln (7) zum Einspritzen (16, 17, 18, 17') von flüssigem $SO_2$ in den Reaktor ausgestattet ist.

9. Verfahren nach Anspruch 8, dadurch gekennzeichnet, dass vor dem Mischer (4) ein Vormischer (4') für das $SO_2$ (2) und das Chlor (3) angeordnet ist.

10. Vorrichtung nach Anspruch 8 oder 9, dadurch gekennzeichnet, dass sie mit einem zusätzlichen Separator (15) ausgestattet ist, der Kühlmittel (15') zur Kondensation von überschüssigem $SO_2$, das vom Reaktor (6) und vom ersten Separator (12) kommt, aufweist.

11. Vorrichtung nach Anspruch 10, dadurch gekennzeichnet, dass sie einen Verdampfer (20) und eine Rückführleitung (19, 19') zur Wiederverwendung eines Teils des verflüssigten $SO_2$ (15, 16) im Vormischer (4') umfasst.

12. Vorrichtung nach einem der Ansprüche 8 bis 11, dadurch gekennzeichnet, dass sie einen Kreislauf (9, 10, 9') zur Rückführung einer Fraktion des im Reaktor gebildeten Rohproduktes in der Zuleitung (5) des Gasgemisches zum Reaktor aufweist.

13. Vorrichtung nach einem der Ansprüche 8 bis 12, dadurch gekennzeichnet, dass der Boden des Reaktors (6) direkt mit einem auf tieferem Niveau befindlichem Speicher (13) über eine Leitung (11a), deren oberer Teil eine Verzweigung (11b) zu einem Gasabscheider (12) aufweist, verbunden ist.

## Claims

1. Process of preparation of alkane sulphonyl halides by the reaction between an alkane, sulphur dioxide and a halogen in the presence of ultraviolet light in the gaseous phase, characterized in that the gaseous mixture of the reactants contains at least 4 moles of $SO_2$ per mole of hydrocarbon

2. Process according to claim 1, characterised in that the temperature in the reaction zone is regulated by the injection of liquid $SO_2$ into the reacting gaseous mixture.

3. Process according to claim 1 or 2, characterised in that the reaction takes place at a temperature from 10° to 90°C, under a pressure of 0.1 to 30 bars, such that the reacting mixture remains gaseous.

4. Process according to claim 3, characterised in that the temperature ranges from 30° to 70°C and the relative pressure from 7 to 15 bars.

5. Process according to any of the preceding claims, in which the alkane is methane, the temperature being from 50° to 70°C.

6. Process according to any of the preceding claims, characterised in that the reaction mixture contains, per mole of methane, 4 to 8 moles of $SO_2$ and 0.6 to 1 mole of $Cl_2$ or, per mole of another gaseous alkane, 7 to 14 moles of $SO_2$ and 0.6 to 1 mole of $Cl_2$.

7. Process according to claim 6, characterised in that the proportions, per mole of methane, are 5 to 7 moles of $SO_2$ and 0.7 to 0.9 mole of $Cl_2$ or, per mole of another gaseous alkane, 10 to 13 moles of $SO_2$ and 0.7 to 0.9 mole of $Cl_2$.

8. Apparatus for carrying out the process according to any of claims 1 to 7, which comprises a mixer (4) for the gaseous reactants supplying a reactor (6) comprising an ultraviolet lamp (8) and separators (12, 13) downstream from the reactor (6), characterised in that the reactor (6) is provided with means (7) for the injection (16, 17, 18, 17') of liquid $SO_2$ into the reactor.

9. Apparatus according to claim 8, characterised in that the mixture (4) is preceded by a premixer (4') for the $SO_2$ (2) and the chlorine (3).

10. Apparatus according to claim 8 or 9, characterised in that a supplementary separator (15) is provided, having cooling means (15') for the condensation of excess $SO_2$, derived from the reactor (6) and from the first separator (12).

11. Apparatus according to claim 10, characterised in that it comprises an evaporator (20) and a recycling circuit (19, 19') for reusing, in the premixer (4'), a part of the liquefied $SO_2$ (15, 16).

12. Apparatus according to any of claims 9 to 11, characterised in that it comprises a circuit (9, 10, 9') for reintroducing a fraction of the crude product formed in the reactor into the conduit (5) supplying the gaseous mixture to the reactor.

13. Apparatus according to any of claims 8 to 12, characterised in that the base of the reactor (6) is connected directly to a reservoir (13) situated at a lower level by a duct (11a), the upper part of which carries a side branch (11b) directed to a gas separator (12).

# FIG_1

FIG. 2